# EUROPÄISCHE PATENTSCHRIFT

(11) **EP 3 821 201 B1**
(45) Veröffentlichungstag und Bekanntmachung des Hinweises auf die Patenterteilung: **22.03.2023**
(21) Anmeldenummer: 19731640.9
(22) Anmeldetag: 11.06.2019
(51) Int. Cl.: G01B 11/24, G01B 5/00, G01B 11/25, A61B 1/00, A61B 1/24, A61B 5/00, A61C 9/00, G06T 7/55, H04N 13/221, A61B 5/107

(54) **OPTISCHES MESSVERFAHREN SOWIE OPTISCHE MESSVORRICHTUNG**
OPTICAL MEASURING METHOD AND OPTICAL MEASURING DEVICE
PROCÉDÉ DE MESURE OPTIQUE AINSI QUE DISPOSITIF DE MESURE OPTIQUE

(30) Priorität: 10.07.2018 DE 102018211369
(43) Veröffentlichungstag der Anmeldung: 19.05.2021
(73) Patentinhaber: Sirona Dental Systems GmbH, 64625 Bensheim (DE); Dentsply Sirona Inc., York, PA 17401-2991 (US)
(72) Erfinder: KLEIN, Konrad, 69121 Heidelberg (DE); FRITZ, Peter, 68163 Mannheim (DE); ADAMSON, Anders, 64297 Darmstadt (DE)
(74) Vertreter: Özer, Alpdeniz
(86) Internationale Anmeldenummer: PCT/EP2019/065147
(87) Internationale Veröffentlichungsnummer: WO 2020/011464

(56) Entgegenhaltungen:
- EP-A1- 3 090 680
- WO-A1-2017/062044
- US-A1- 2014 272 765
- US-A1- 2018 140 394

## Beschreibung

### Technisches Gebiet

Die Erfindung betrifft ein optisches Messverfahren zur dreidimensionalen Erfassung der Oberfläche eines Objekts mit einer optische Aufnahmeeinheit, wobei während eines ersten Messzeitintervalls die optische Aufnahmeeinheit relativ zu dem Objekt bewegt wird, das Objekt von der Aufnahmeeinheit mit einem Beleuchtungsstrahl mit einer Lichtintensität beleuchtet wird und von der Aufnahmeeinheit mit einer Aufnahmefrequenz nacheinander Höhenbilder erfasst werden, wobei erfasste Höhenbilder noch während des Messzeitintervalls zu einem Gesamthöhenbild hinzugefügt werden und das Gesamthöhenbild angezeigt wird.

### Stand der Technik

Aus der EP 2 172 799 A1 ist eine optische Messvorrichtung bekannt, die eine dreidimensionale optische Erfassung eines Objekts mittels eines konfokalen Abbildungssystems ermöglicht. Ebenfalls konfokal und zusätzlich mit einem auf das Objekt projizierten, bewegten Muster arbeitet die aus der WO 2015/036467 bekannte optische Messvorrichtung.

Zur Vermessung von Objekten, deren Größe die Größe des Aufnahmebereichs der Messvorrichtung überschreitet, werden mehrere Einzelaufnahmen erzeugt und zu einem Gesamtbild zusammengefügt. Die Einzelaufnahmen werden zeitlich nacheinander erzeugt, während Messvorrichtung und Objekt relativ zueinander bewegt werden. Für das Zusammenfügen der Einzelaufnahmen müssen die relativen Orientierungen der Einzelaufnahmen zueinander bestimmt werden. Dieser Schritt wird als Registrierung bezeichnet. Algorithmen zum Registrieren der Bilddaten sind beispielsweise aus "A Method for Registration of 3-D Shapes" von Besl et al., IEEE Transactions on pattern analysis and machine intelligence, Vol. 14, No. 2, 1992 oder aus "Multiview Registration of Large Data Sets" von Pulli, Proceedings, Second International Conference on 3D Digital Imaging and Modeling, Ottawa 1999, pp. 160-168 bekannt.

Sowohl das Erzeugen von Projektionsmustern oder von Licht zur Beleuchtung des Objekts im Allgemeinen als auch das Registrieren und Übertragen der einzelnen Datensätze benötigt Energie, wobei die durch den Energieverbrauch entstehende Abwärme insbesondere für intraoral zu verwendende Messvorrichtungen, z.B. Intraoral-Kameras, unerwünscht ist.

Eine Möglichkeit, die Wärmeentwicklung zu reduzieren bzw. eine unnötige Wärmeentwicklung zu vermeiden, besteht darin, das Erzeugen von unnötigen Bilddatensätzen, also von für das Gesamtbild nicht benötigten Bilddatensätzen, zu vermeiden.

Zur Vermeidung unnötiger Aufnahmen ist es beispielsweise bekannt, den Anwender während des Aufnahmezeitintervalls durch Feedback zu leiten. Beispielsweise ist aus der DE 10 2014 207 667 A1 bekannt, dem Anwender während der Aufnahme bereits erfasste Bereiche des Objekts in einem Standardmodell anzuzeigen. Der Anwender kann entsprechend reagieren und muss die bereits ausreichend vermessenen Bereiche nicht nochmals vermessen.

US2018/140394A1 offenbart einen digitalen dentalen Löffel. In der US 2014/0272765 A1 und in der WO 2017/062044 A1 sind verschiedene Regelsystems für 3D-Sensoren offenbart.Vor diesem Hintergrund besteht die Aufgabe der vorliegenden Anmeldung darin, die bekannten Messvorrichtungen und Messverfahren zu verbessern und insbesondere den Energieverbrauch und Rechenaufwand in zuverlässiger und möglichst von dem Anwender unabhängiger Weise zu reduzieren.

### Darstellung der Erfindung

Die Aufgabe wird durch ein optisches Messverfahren gemäß Anspruch 1 und ein optisches Messsystem gemäß Anspruch 10 gelöst. Vorteilhafte Weiterbildungen sind in den abhängigen Ansprüchen angegeben.

Ein Gegenstand der Erfindung ist ein optisches Messverfahren zur dreidimensionalen Erfassung der Oberfläche eines Objekts mit einer optischen Aufnahmeeinheit sowie ein zur Ausführung des optischen Messverfahrens ausgelegtes optisches Messsystem mit einer optischen Aufnahmeeinheit, einer computerlesbaren Speichereinheit, einer Recheneinheit und einer Anzeigeeinheit.

Während eines ersten Messzeitintervalls wird die optische Aufnahmeeinheit relativ zu dem Objekt bewegt, wobei das Objekt von der Aufnahmeeinheit mit einem Beleuchtungsstrahl mit einer Lichtintensität beleuchtet wird und von der Aufnahmeeinheit mit einer Aufnahmefrequenz nacheinander Höhenbilder erfasst werden. Zumindest ein Teil der erfassten Höhenbilder wird während des Messzeitintervalls zu einem Gesamthöhenbild hinzugefügt und angezeigt.

Die Lichtintensität und/oder die Aufnahmefrequenz werden während des Messzeitintervalls durch Steuersignale geregelt. Die Steuersignale werden während des Messzeitintervalls in zeitlichen Abständen erzeugt, wobei jedes Steuersignal auf Basis mindestens eines Sensorsignals mindestens eines Temperatursensors erzeugt wird.

Ein weiterer Gegenstand der Erfindung ist ein optisches Messsystem, aufweisend eine optische Aufnahmeeinheit, eine computerlesbare Speichereinheit, eine Recheneinheit und eine Anzeigeeinheit, wobei das optische Messsystem dazu ausgelegt ist, das hier beschriebene optische Messverfahren auszuführen.

Als Höhenbild wird eine Pixelmatrix oder Bildmatrix bezeichnet, wobei jeder Pixel bzw. Bildpunkt eine dreidimensionale Information enthält, nämlich die dreidimensionale Position der Objektoberfläche bzw. die Höhe der Objektoberfläche für den jeweiligen Bildpunkt im Aufnahmebereich. Die dreidimensionalen Informationen sind beispielsweise aus einer Bildsequenz, z.B. mittels Phase-Shift-Triangulation, extrahiert worden. Das Gesamthöhenbild wird aus den vielen, während des Messzeitintervalls aufgenommenen Höhenbildern zusammengesetzt, so dass auch Objekte vermessen werden können, deren Größe die Größe des Aufnahmebereichs der Aufnahmeeinheit übersteigt.

Als erstes Messzeitintervall wird eine Zeitspanne zwischen einem Einschalten der optischen Aufnahmeeinheit und einem Ausschalten der optischen Aufnahmeeinheit bezeichnet.

Es versteht sich, dass die Aufnahmefrequenz sowie die Lichtintensität grundsätzlich beliebige Werte annehmen können. Entsprechend kann die Beleuchtung beispielsweise während des Messzeitintervalls zeitweise ausgeschaltet werden, d.h. die Lichtintensität auf Null reduziert werden. Ebenso kann es während des Messzeitintervalls regelmäßige Totzeiten geben, während derer die Lichtintensität Null beträgt.

Die Lichtintensität wird beispielsweise über den Strom oder über den duty cycle bzw. den Tastgrad, also das Verhältnis von Impulsdauer zu Periodendauer, geregelt.

Ein Vorteil des erfindungsgemäßen Verfahrens sowie der erfindungsgemäßen Vorrichtung ist eine zuverlässige und einfache Kontrolle der Wärmeentwicklung der Aufnahmeeinheit.

Vorteilhafterweise wird das Steuersignal zusätzlich auf Basis mindestens eines Parameters der Aufnahmeeinheit und/oder mindestens eines bereits erfassten Höhenbilds erzeugt.

Werden die Aufnahmefrequenz sowie die Lichtintensität der Beleuchtung möglichst gering gehalten, so kann Energie und/oder Rechenaufwand eingespart und eine unnötige Wärmeentwicklung vermieden werden. Andererseits ist eine gewisse Beleuchtungsintensität bzw. Aufnahmefrequenz notwendig, um einen Aufnahmebereich zu erfassen und eine gewisse Qualität zu erhalten. Auch die Übertragung von Aufnahmedaten von der Aufnahmeeinheit zu einer Recheneinheit kann zur Einsparung von Energie und Einschränkung der Wärmeentwicklung zeitweise ausgesetzt werden.

Durch Erfassen eines Zustands des Messverfahrens während der Messung ist es möglich, die Aufnahmefrequenz und/oder die Lichtintensität zeitnah an den aktuellen Zustand anzupassen und hierdurch einerseits eine ausreichende Qualität und andererseits einen möglichst geringen Energieverbrauch und/oder Rechenaufwand sicherzustellen.

Der Zustand des Messverfahrens wird während des Messzeitintervalls mittels eines oder mehrerer zusätzlicher Sensoren oder anhand von Parametern der Aufnahmeeinheit oder anhand von bisher erfassten Daten oder anhand einer Kombination der vorgenannten Alternativen ermittelt. Der Zustand kann jede für das Messverfahren bzw. das Gesamtbild relevante Größe sein, z.B. eine Abtastdichte, eine Geschwindigkeit der Aufnahmeeinheit oder eine Größe eines Überlapps von aufeinanderfolgenden Bildern oder eine Temperatur der optischen Aufnahmeeinheit. Die Aufnahmefrequenz und/oder Beleuchtungsintensität wird dann an den ermittelten Zustand angepasst.

Es versteht sich, dass für jedes Steuersignal jeweils möglichst aktuelle Werte des Temperatursensors sowie des zusätzlichen Sensors und/oder der Aufnahmeeinheit und/oder das zuletzt aufgenommene Höhenbild herangezogen werden, um möglichst den aktuellen Zustand der Aufnahmeeinheit und/oder der aufgenommenen Daten zu berücksichtigen.

Zur Erzeugung der Steuersignale steht beispielsweise eine Recheneinheit zur Verfügung, welche mit der optischen Aufnahmeeinheit über ein Kabel oder kabellos kommuniziert. Alternativ kann eine Recheneinheit in der optischen Aufnahmeeinheit integriert sein.

Die Steuersignale werden in festen zeitlichen Abständen, also mit einer festen Frequenz, erzeugt. Alternativ werden die Steuersignale in unregelmäßigen zeitlichen Abständen erzeugt, beispielsweise aufgrund eines Triggersignals. Gemäß einer weiteren alternativen Ausführungsform wird zu jedem erfassten Höhenbild ein zumindest zeitlich zugehöriges Steuersignal erzeugt, so dass sich der zeitliche Abstand der Steuersignalerzeugung nach der Aufnahmefrequenz der Höhenbilder richtet.

Die Regelung der Aufnahmefrequenz in Abhängigkeit von einem aktuellen Zustand der Aufnahme ermöglicht es, einerseits das Aufnehmen von überflüssigen Daten zu vermeiden und andererseits sicherzustellen, dass noch ausreichend Daten erfasst werden, um ein Gesamthöhenbild zu erzeugen.

Befindet sich die optische Aufnahmeeinheit beispielsweise über noch nicht erfassten Bereichen oder über bisher nur lückenhaft erfassten Bereichen, wird die Aufnahmefrequenz erhöht. Andererseits wird die Aufnahmefrequenz verringert, wenn sich die optische Aufnahmeeinheit über bereits vollständig oder fast vollständig erfassten Bereichen befindet. Das Maß der Erfassung wird beispielsweise anhand des Überlapps des zuletzt aufgenommenen Höhenbilds mit vorher aufgenommenen Höhenbildern und/oder aus Bewegungs- und/oder Positionsdaten der Aufnahmeeinheit ermittelt. Bewegungs- und/oder Positionsdaten werden z.B. aus einem oder mehreren erfassten Höhenbildern oder einem oder mehreren Sensorsignalen eines bzw. mehrerer entsprechender Sensoren ermittelt. Alternativ zusätzlich zu Bewegungssensoren und/oder Temperatursensoren kann erfindungsgemäß auch eine Farbbildkamera als Sensor eingesetzt werden.

Durch die Regelung der Lichtintensität wird diese ebenfalls während des Messzeitintervalls an den aktuellen Zustand der Aufnahme angepasst. Hierdurch kann die Lichtintensität, wann immer möglich, reduziert werden, wobei eine ausreichende Qualität der Daten sichergestellt wird.

Beispielsweise werden das zuletzt erfasste Höhenbild oder mehrere bereits erfasste Höhenbilder oder das bisher erzeugte Gesamthöhenbild hinsichtlich einer Oberflächenbeschaffenheit des darin enthaltenen Objektbereichs analysiert. Weist der aktuell im Aufnahmebereich befindliche Teil des Objekts beispielsweise steile Kanten auf, so wird die Lichtintensität erhöht, während bei einer glatteren Topologie die Lichtintensität reduziert wird.

Ebenso kann eine Anpassung der Aufnahmefrequenz und/oder der Lichtintensität an den Zustand der Aufnahmeeinheit erfolgen. Beispielsweise werden die Aufnahmefrequenz und/oder die Lichtintensität reduziert, sobald ein oder mehrere Parameter der Aufnahmeeinheit ein unerwünschtes Aufheizen der Aufnahmeeinheit anzeigen. Die Aufnahmefrequenz kann beispielsweise auch reduziert werden, wenn ein durch die Höhenbilder und/oder das Gesamthöhenbild gebildetes Datenvolumen einen vorgebbaren oder vorgegebenen Grenzwert überschreitet oder eine das Gesamthöhenbild erzeugende Recheneinheit überlastet ist.

Ein Vorteil ist daher ein reduzierter Energieverbrauch, wodurch insbesondere auch ein Erwärmen der Aufnahmeeinheit reduziert wird. Gleichzeitig wird sichergestellt, dass die Qualität der Aufnahmen bzw. des Gesamthöhenbilds sich nicht reduziert.

Vorzugsweise wird zur Erzeugung jedes Steuersignals zu dem jeweils zuletzt erfassten Höhenbild eine Maßzahl eines Überlapps des zuletzt erfassten Höhenbilds mit einem unmittelbar vorausgehend erfassten Höhenbild und/oder eine Maßzahl eines Überlapps des zuletzt erfassten Höhenbilds mit dem Gesamthöhenbild bestimmt und ein der Maßzahl entsprechendes Steuersignal erzeugt.

Typischerweise wird für das Registrieren des jeweils neuen Höhenbilds mit den bereits erfassten Höhenbildern, also dem Ermitteln der Ausrichtung bzw. Orientierung des neuen Höhenbilds relativ zu den bereits erfassten Höhenbildern, ein Überlapp des zuletzt erfassten Höhenbilds mit dem unmittelbar vorhergehend erfassten Höhenbild oder dem Gesamthöhenbild bestimmt. Der Überlapp eignet sich auch als Maß für die Menge der für einen Objektbereich bereits vorhandenen Daten sowie zur Bestimmung einer Geschwindigkeit und/oder Bewegungsrichtung der optischen Aufnahmeeinheit relativ zu dem Objekt.

Gemäß einer Weiterbildung wird bei dem Bestimmen des Überlapps zusätzlich das Sensorsignal des mindestens einen Sensors berücksichtigt. Es ist insbesondere vorteilhaft, eine Auswertung des Überlapps, also des Fortgangs der Erfassung mit einer Temperaturkontrolle, also einem die Temperatur der optischen Aufnahmeeinheit oder der Recheneinheit erfassenden Sensor, zu kombinieren.

Alternativ oder ergänzend wird zur Erzeugung jedes Steuersignals zu dem jeweils zuletzt erfassten Höhenbild ein Oberflächenzuwachs einer im Gesamthöhenbild enthaltenen Gesamtoberfläche durch eine im zuletzt erfassten Höhenbild enthaltene Oberfläche bestimmt und ein dem Oberflächenzuwachs entsprechendes Steuersignal erzeugt. Der Oberflächenzuwachs eignet sich ebenfalls als Maß für die Menge der für einen Objektbereich bereits vorhandenen Daten sowie zur Bestimmung einer Geschwindigkeit und/oder einer Bewegungsrichtung der optischen Aufnahmeeinheit.

Die erfassten Höhenbilder bzw. das aus den Höhenbildern zusammengefügte Gesamthöhenbild sind auf unterschiedliche Weisen darstellbar. Vorteilhaft ist es beispielsweise, eine Objektoberfläche innerhalb des Volumendatensatzes als Dreiecksnetz zu identifizieren bzw. darzustellen. Die Oberfläche kann beispielsweise gemäß dem in "Marching Cubes: A High Resolution 3D Surface Construction Algorithm", W. E. Lorensen und H. E. Cline, ACM SIGGRAPH Computer Graphics, Vol. 21, Nr. 4, pp. 163-169, August 1987 beschriebenen Verfahren dargestellt werden. Eine solche Darstellung der Objektoberfläche ermöglicht eine besonders einfache und damit auch besonders Energie-effiziente Bestimmung des Oberflächenzuwachses.

Vorteilhafterweise wird aus der Maßzahl des Überlapps oder aus dem Oberflächenzuwachs eine Abtastdichte oder eine Kamerageschwindigkeit der optischen Aufnahmeeinheit bestimmt und zur Erzeugung des Steuersignals verwendet.

Alternativ oder ergänzend werden zur Erzeugung jedes Steuersignals für das jeweils zuletzt erfasste Höhenbild die Gesamtintensität und/oder die maximale Intensität und/oder der Kontrast und/oder die Anzahl von extrahierten Datenpunkten und/oder eine Qualität von extrahierten Datenpunkten und/oder das Signal/Rausch-Verhältnis und/oder der Kontrast eines zusätzlich erzeugten Farbbilds bestimmt und zur Erzeugung des Steuersignals verwendet. Als extrahierte Datenpunkte werden alle von der Aufnahmeeinheit erfassten Pixel bzw. Bildpunkte bezeichnet, die für ein Höhenbild verwendet werden.

Vorteilhafterweise wird der Parameter der Aufnahmeeinheit mit einem weiteren Sensor erfasst, wobei der Parameter eine Geschwindigkeit der optischen Aufnahmeeinheit und/oder eine Beschleunigung der optischen Aufnahmeeinheit und/oder eine räumliche Position der optischen Aufnahmeeinheit und/oder eine Bewegung der optischen Aufnahmeeinheit ist.

Vorteilhafterweise wird vor dem Hinzufügen eines Höhenbilds zu dem Gesamthöhenbild ein Registrierverfahren für das Hinzufügen in Abhängigkeit von der Aufnahmefrequenz ausgewählt.

### Kurzbeschreibung der Zeichnungen

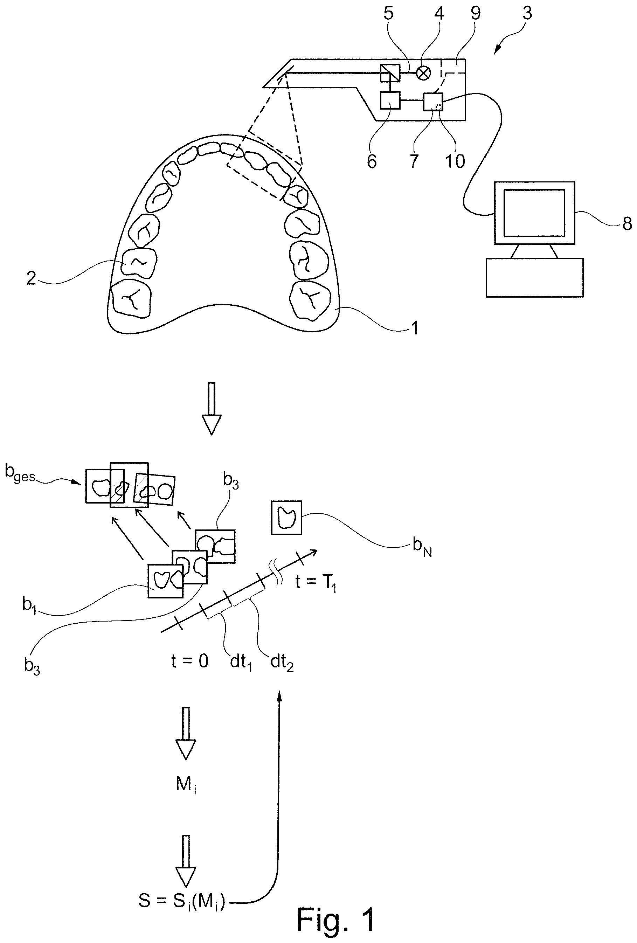

Ausführungsbeispiele der Erfindung sind in der Zeichnung dargestellt. Es zeigt die
- Fig. 1: eine schematische Darstellung einer ersten Ausführungsform eines erfindungsgemäßen Aufnahmeverfahrens.

### Ausführungsbeispiele

In Fig. 1 ist eine erste Ausführungsform eines erfindungsgemäßen Aufnahmeverfahrens schematisch veranschaulicht.

Gemäß dem Ausführungsbeispiel wird ein Unterkiefer 1 mit Zähnen 2 als Objekt mittels einer optische Aufnahmeeinheit 3 vermessen. Die optische Aufnahmeeinheit 3 ist als intraorale Kamera ausgebildet und umfasst eine Lichtquelle 4 und einen Lichtdetektor 6 und ist mit einer Recheneinheit 7 mit einem Anzeigemittel 8 verbunden.

Die Vermessung wird während eines Zeitintervalls T1 durchgeführt, wobei die intraorale Kamera 3 über die Zähne 2 des Unterkiefers 1 und/oder Fehlstellen im Unterkiefer 1 bewegt wird. Die Lichtquelle 4 stellt einen Beleuchtungsstrahl 5 bereit und der Lichtdetektor 6 detektiert reflektiertes Licht. Die Erfassung des reflektierten Lichts erfolgt mit einer Aufnahmefrequenz f_{A}, wobei mit einem zeitlichen Abstand dt=1/f_{A} jeweils ein Datensatz von dem Lichtdetektor 6 erfasst wird und an die Recheneinheit übermittelt wird. Die Recheneinheit berechnet zu jedem Datensatz jeweils ein Höhenbild bᵢ, i=1...N und speichert dieses in einem Speichermedium der Recheneinheit 8 ab. Die Aufnahmefrequenz f_{A} ist veränderbar, so dass die zeitlichen Abstände dt zwischen aufeinanderfolgend aufgenommenen Höhenbildern bᵢ nicht unbedingt gleich sind.

Die erzeugten Höhenbilder bᵢ werden bereits während des Messzeitintervalls T1 nach und nach zu einem Gesamthöhenbild b_{ges} zusammengefügt, wobei das Gesamthöhenbild b_{ges} bereits während des Entstehens mittels des Anzeigemittels 8 angezeigt wird. Es versteht sich, dass gegebenenfalls nicht alle erzeugten Höhenbilder für das Gesamthöhenbild verwendet werden, sondern dass einzelne Höhenbilder beispielsweise aufgrund mangelnder Qualität aussortiert werden. Hierzu wird ein erstes Höhenbild b₁ als Gesamthöhenbild b_{ges} abgespeichert und angezeigt. Anschließend werden kontinuierlich weitere aufgenommene Höhenbilder bᵢ, i=2...N zu dem Gesamthöhenbild b_{ges} hinzugefügt und das neue Gesamthöhenbild b_{ges} angezeigt, wobei eine relative Ausrichtung des Höhenbilds bᵢ zu dem Gesamthöhenbild b_{ges} anhand eines Überlapps (schraffiert dargestellt) des Höhenbilds bᵢ mit dem Gesamthöhenbild b_{ges}, also den bisher aufgenommenen Höhenbildern bᵢ, insbesondere dem unmittelbar vorangegangen aufgenommenen Höhenbild bᵢ₋₁, ermittelt wird.

Darüber hinaus wird die Aufnahmefrequenz f_{A} im dargestellten Ausführungsbeispiel anhand des ermittelten Überlapps geregelt. Zu dem Überlapp des zuletzt aufgenommenen Höhenbilds bᵢ mit dem Gesamthöhenbild b_{ges} wird jeweils eine erste Maßzahl Mᵢ, z.B. die flächenmäßige Größe des Überlapps, bestimmt und anhand der ersten Maßzahl Mᵢ ein erstes Steuersignal S = Sᵢ(Mᵢ) zur Steuerung der Aufnahmefrequenz f_{A} erzeugt. Die Aufnahmefrequenz f_{A} des Lichtdetektors 6 wird anschließend mittels einer Steuereinheit 10, die im dargestellten Ausführungsbeispiel Teil der optischen Aufnahmeeinheit 3 ist, mittels des Steuersignals S geregelt, also gegebenenfalls verändert. Das nächste Höhenbild bᵢ₊₁ wird entsprechend mit einem zeitlichen Abstand dtᵢ₊₁=1/f_{A} erfasst, wobei f_{A} die geregelte Aufnahmefrequenz bezeichnet.

Ferner wird anhand des zuletzt aufgenommenen Höhenbilds bᵢ eine zweite Maßzahl für eine Qualität des Höhenbildes bᵢ, z.B. eine Gesamtintensität oder ein Kontrast, bestimmt. Anhand der zweiten Maßzahl wird ein zweites Steuersignal zur Steuerung der Lichtintensität des Beleuchtungsstrahls 5 erzeugt und die Lichtintensität entsprechend bzw. mittels des zweiten Steuersignals geregelt.

In einer alternativen Ausführungsform oder ergänzend wird ein Sensorsignal eines Sensors 9 (gestrichelt dargestellt) herangezogen, um das Steuersignal S zu berechnen. Beispielsweise wird die Bewegung der intraoralen Kamera 3 mittels eines integrierten Inertialmesssystems nachvollzogen, wobei aus der Bewegung der Kamera 4 auf die Ausrichtung der einzelnen Höhenbilder bᵢ zueinander geschlossen werden kann.

### Bezugszeichenliste

- 1: Objekt
- 2: Zahn
- 3: Aufnahmeeinheit
- 4: Lichtquelle
- 5: Beleuchtungsstrahl
- 6: Lichtdetektor
- 7: Recheneinheit
- 8: Anzeigeeinheit
- 9: Sensor
- 10: Steuereinheit
- b_{ges}: Gesamthöhenbild
- bᵢ: Höhenbilder
- f_{A}: Aufnahmefrequenz
- dt: zeitlicher Abstand
- Mᵢ: Maßzahl
- S: Steuersignal
- T1: Aufnahmezeitintervall

## Patentansprüche

1. Optisches Messverfahren zur dreidimensionalen Erfassung der Oberfläche eines Objekts (1) mit einer optischen Aufnahmeeinheit (3), wobei
- während eines ersten Messzeitintervalls (T1) die optische Aufnahmeeinheit (3) relativ zu dem Objekt (1) bewegt wird,
- während des ersten Messzeitintervalls (T1) das Objekt (1) von der Aufnahmeeinheit (3) mit einem Beleuchtungsstrahl (5') mit einer Lichtintensität (I) beleuchtet wird,
- während des ersten Messzeitintervalls (T1) von der Aufnahmeeinheit (3) mit einer Aufnahmefrequenz (f_{A}) nacheinander Höhenbilder (bᵢ) erfasst werden,
- zumindest ein Teil der erfassten Höhenbilder (bᵢ) während des Messzeitintervalls (T1) jeweils zu einem Gesamthöhenbild (b_{ges}) hinzugefügt und das Gesamthöhenbild (b_{ges}) angezeigt wird,
**dadurch gekennzeichnet, dass**
- die Lichtintensität(I) und/oder die Aufnahmefrequenz (f_{A}) während des Messzeitintervalls (T1) durch Steuersignale (S) geregelt werden, wobei
- die Steuersignale (S) während des Messzeitintervalls (T1) in zeitlichen Abständen erzeugt werden und
- jedes Steuersignal (S) auf Basis mindestens eines Sensorsignals (10) von mindestens einem Temperatursensor (9) erzeugt wird.

2. Optisches Messverfahren nach Anspruch 1, **dadurch gekennzeichnet, dass** das Steuersignal (S) zusätzlich auf Basis mindestens eines Parameters der Aufnahmeeinheit (3) und/oder mindestens eines bereits erfassten Höhenbilds (bᵢ) erzeugt wird.

3. Optisches Messverfahren nach Anspruch 2, **dadurch gekennzeichnet, dass** zur Erzeugung jedes Steuersignals (S) zu dem jeweils zuletzt erfassten Höhenbild (bᵢ)
- eine Maßzahl (Mᵢ) eines Überlapps des zuletzt erfassten Höhenbilds mit einem unmittelbar vorausgehend erfassten Höhenbild (bᵢ₋₁) und/oder
- eine Maßzahl (Mᵢ) eines Überlapps des zuletzt erfassten Höhenbilds mit dem Gesamthöhenbild (b_{ges})
bestimmt und anschließend ein der Maßzahl (Mᵢ) entsprechendes Steuersignal (S) erzeugt wird.

4. Optisches Messverfahren nach Anspruch 3, **dadurch gekennzeichnet, dass** bei dem Bestimmen der Maßzahl (Mᵢ) des Überlapps das Sensorsignal (10) des mindestens einen Temperatursensors (9) berücksichtigt wird.

5. Optisches Messverfahren nach Anspruch 2, **dadurch gekennzeichnet, dass** zur Erzeugung jedes Steuersignals (S) zu dem jeweils zuletzt erfassten Höhenbild (bᵢ) ein Oberflächenzuwachs einer im Gesamthöhenbild (b_{ges}) enthaltenen Gesamtoberfläche durch eine in dem zuletzt erfassten Höhenbild enthaltene Oberfläche bestimmt und ein dem Oberflächenzuwachs entsprechendes Steuersignal (S) erzeugt wird.

6. Optisches Messverfahren nach einem der Ansprüche 3 bis 5, **dadurch gekennzeichnet, dass** aus der Maßzahl (Mᵢ) des Überlapps oder aus dem Oberflächenzuwachs eine Abtastdichte oder eine Geschwindigkeit der optischen Aufnahmeeinheit (3) bestimmt und zur Erzeugung des Steuersignals (S) verwendet wird.

7. Optisches Messverfahren nach einem der Ansprüche 2 bis 5, **dadurch gekennzeichnet, dass** zur Erzeugung jedes Steuersignals (S) für das jeweils zuletzt erfasste Höhenbild (bᵢ) die Gesamtintensität und/oder die maximale Intensität und/oder der Kontrast und/oder die Anzahl von extrahierten Datenpunkten und/oder eine Qualität von extrahierten Datenpunkten und/oder das Signal/Rausch-Verhältnis und/oder der Kontrast eines zusätzlich erzeugten Farbbilds bestimmt und zur Erzeugung des Steuersignals verwendet wird.

8. Optisches Messverfahren nach einem der Ansprüche 2 bis 6, dadurch gegenzeichnet, dass der Parameter der Aufnahmeeinheit (3) mit einem weiteren Sensor erfasst wird, wobei der Parameter eine Geschwindigkeit der optischen Aufnahmeeinheit (3) und/oder eine Beschleunigung der optischen Aufnahmeeinheit (3) und/oder einen räumliche Position der optischen Aufnahmeeinheit (3) und/oder eine Bewegung der optischen Aufnahmeeinheit (3) ist.

9. Optisches Messverfahren nach einem der Ansprüche 1 bis 7, **dadurch gekennzeichnet, dass** vor dem Hinzufügen eines Höhenbilds (bᵢ) zu dem Gesamthöhenbild (b_{ges}) ein Registrierverfahren für das Hinzufügen in Abhängigkeit von der Aufnahmefrequenz (f_{A}) ausgewählt wird.

10. Optisches Messsystem, aufweisend eine optische Aufnahmeeinheit, eine computerlesbare Speichereinheit, eine Recheneinheit und eine Anzeigeeinheit, **dadurch gekennzeichnet, dass** die optische Aufnahmeeinheit eine Lichtquelle, einen Lichtdetektor, einen Temperatursensor, und eine Steuereinheit die die Steuersignale erzeugt umfasst, und das optische Messsystem dazu ausgelegt ist, das optische Messverfahren nach einem der Ansprüche 1 bis 9 auszuführen.

## Claims

1. An optical measuring method for three-dimensional acquisition of the surface of an object (1) with an optical capture unit (3), wherein,
- during a first measurement time interval (T1), the optical capture unit (3) is moved relative to the object (1),
- during the first measurement time interval (T1), the object (1) is illuminated by the capture unit (3) with an illumination beam (5') with a light intensity (I),
- during the first measurement time interval (T1), height images (bᵢ) are successively acquired by the capture unit (3) at a capture frequency (f_{A}),
- at least some of the acquired height images (bᵢ) being added to a total height image (bₜₒₜ) during the measurement time interval (T1) and the total height image (bₜₒₜ) being displayed,
**characterised in that**
- the light intensity (I) and/or the capture frequency (f_{A}) during the measurement time interval (T1) is/are controlled by control signals (S), wherein
- the control signals (S) are generated at time intervals during the measurement time interval (T1) and
- each control signal (S) is generated on the basis of at least one sensor signal (10) from at least one temperature sensor (9).

2. The optical measuring method according to claim 1, **characterised in that** the control signal (S) is additionally generated on the basis of at least one parameter of the capture unit (3) and/or at least one already acquired height image (bᵢ).

3. The optical measuring method according to claim 2, **characterised in that** for the generation of each control signal (S) for the last acquired height image (bᵢ),
- a measured value (Mᵢ) of an overlap of the last acquired height image with a height image (bᵢ₋₁) acquired immediately beforehand and/or
- a measured value (Mᵢ) of an overlap of the last acquired height image with the total height image (bₜₒₜ) is/are determined and then a control signal (S) corresponding to the measured value (Mᵢ) is generated.

4. The optical measuring method according to claim 3, **characterised in that** the sensor signal (10) of the at least one temperature sensor (9) is taken into account when the measured value (Mᵢ) of the overlap is determined.

5. The optical measuring method according to claim 2, **characterised in that** for the generation of each control signal (S) for the last acquired height image (bᵢ), a surface increase of a total surface contained in the total height image (bₜₒₜ) is determined by means of a surface contained in the last acquired height image and a control signal (S) corresponding to the surface increase is generated.

6. The optical measuring method according to one of claims 3 to 5, **characterised in that** a scanning density or a speed of the optical capture unit (3) is determined from the measured value (Mᵢ) of the overlap or from the increase in surface area and is used to generate the control signal (S).

7. The optical measuring method according to one of claims 2 to 5, **characterised in that** to generate each control signal (S) for the last acquired height image (bᵢ), the total intensity and/or the maximum intensity and/or the contrast and/or the number of extracted data points and/or a quality of extracted data points and/or the signal/noise ratio and/or the contrast of an additionally generated colour image is/are determined and used to generate the control signal.

8. The optical measuring method according to one of claims 2 to 6, **characterised in that** the parameter of the capture unit (3) is acquired with a further sensor, wherein the parameter is a speed of the optical capture unit (3) and/or an acceleration of the optical capture unit (3) and/or a spatial position of the optical capture unit (3) and/or a movement of the optical capture unit (3).

9. The optical measurement method according to one of claims 1 to 7, **characterised in that** before a height image (bᵢ) is added to the total height image (bₜₒₜ), a registration method for the addition is selected as a function of the capture frequency (f_{A}).

10. An optical measuring system comprising an optical capture unit, a computer-readable storage unit, a computing unit and a display unit, **characterised in that** the optical capture unit comprises a light source, a light detector, a temperature sensor, and a control unit which generates the control signals, and the optical measuring system is designed to carry out the optical measuring method according to one of claims 1 to 9.

## Revendications

1. Procédé de mesure optique permettant la détection tridimensionnelle de la surface d'un objet (1) comportant une unité d'enregistrement (3) optique, dans lequel
- pendant un premier intervalle temporel de mesure (T1), l'unité d'enregistrement (3) optique est déplacée par rapport à l'objet (1),
- pendant le premier intervalle temporel de mesure (T1), l'objet (1) est éclairé par l'unité d'enregistrement (3) à l'aide d'un faisceau d'éclairage (5') d'une intensité lumineuse (I),
- pendant le premier intervalle temporel de mesure (T1), des images de hauteur (bᵢ) sont successivement enregistrées par l'unité d'enregistrement (3) à une fréquence d'enregistrement (f_{A}),
- au moins une partie des images de hauteur (bᵢ) enregistrées est respectivement ajoutée, pendant l'intervalle temporel de mesure (T1), à une image de hauteur totale (bₜₒₜ) et l'image de hauteur totale (bₜₒₜ) est affichée,
**caractérisé en ce que**
- l'intensité lumineuse (I) et/ou la fréquence d'enregistrement (f_{A}) pendant l'intervalle temporel de mesure (T1) peuvent être réglées au moyen de signaux de commande (S), dans lequel
- les signaux de commande (S) sont produits, pendant l'intervalle temporel de mesure (T1), à des intervalles temporels et
- chaque signal de commande (S) est produit sur la base d'au moins un signal de capteur (10) provenant d'au moins un capteur de température (9).

2. Procédé de mesure optique selon la revendication 1, **caractérisé en ce que** le signal de commande (S) est en outre produit sur la base d'au moins un paramètre de l'unité d'enregistrement (3) et/ou d'au moins une image de hauteur (bᵢ) déjà enregistrée.

3. Procédé de mesure optique selon la revendication 2, **caractérisé en ce que** pour produire chaque signal de commande (S) pour la dernière image de hauteur (bᵢ) respectivement enregistrée,
- une mesure (Mᵢ) d'un chevauchement de la dernière image de hauteur enregistrée avec une image de hauteur (bᵢ₋₁) enregistrée immédiatement avant et/ou
- une mesure (Mᵢ) d'un chevauchement de la dernière image de hauteur enregistrée avec l'image de hauteur totale (bₜₒₜ) est déterminée, puis un signal de commande (S) correspondant à la mesure (Mᵢ) est produit.

4. Procédé de mesure optique selon la revendication 3, **caractérisé en ce que** le signal de capteur (10) de l'au moins un capteur de température (9) est pris en compte lors de la détermination de la mesure (Mᵢ) du chevauchement.

5. Procédé de mesure optique selon la revendication 2, **caractérisé en ce que** pour produire chaque signal de commande (S) pour la dernière image de hauteur (bᵢ) respectivement enregistrée, une augmentation de surface d'une surface totale contenue dans l'image de hauteur totale (bₜₒₜ) est déterminée par une surface contenue dans la dernière image de hauteur enregistrée et le signal de commande (S) correspondant à l'augmentation de surface est produit.

6. Procédé de mesure optique selon l'une quelconque des revendications 3 à 5, **caractérisé en ce qu'**à partir de la mesure (Mᵢ) du chevauchement ou de l'augmentation de surface, une densité de balayage ou une vitesse de l'unité d'enregistrement (3) optique est déterminée et utilisée pour produire le signal de commande (S).

7. Procédé de mesure optique selon l'une quelconque des revendications 2 à 5, **caractérisé en ce que** pour produire chaque signal de commande (S) pour la dernière image de hauteur (bᵢ) respectivement enregistrée, l'intensité totale et/ou l'intensité maximale et/ou le contraste et/ou le nombre de points de données extraits et/ou une qualité de points de données extraits et/ou le rapport signal/bruit et/ou le contraste d'une image couleur produite en plus sont déterminés et utilisés pour produire le signal de commande.

8. Procédé de mesure optique selon l'une quelconque des revendications 2 à 6, **caractérisé en ce que** le paramètre de l'unité d'enregistrement (3) est détecté à l'aide d'un autre capteur, le paramètre étant une vitesse de l'unité d'enregistrement (3) optique et/ou une accélération de l'unité d'enregistrement (3) optique et/ou une position spatiale de l'unité d'enregistrement (3) optique et/ou un mouvement de l'unité d'enregistrement (3) optique.

9. Procédé de mesure optique selon l'une quelconque des revendications 1 à 7, **caractérisé en ce que**, avant d'ajouter une image de hauteur (bᵢ) à l'image de hauteur totale (bₜₒₜ), un procédé d'enregistrement est sélectionné, en fonction de la fréquence d'enregistrement (f_{A}), pour l'ajout.

10. Système de mesure optique comprenant une unité d'enregistrement optique, une unité de mémoire lisible par ordinateur, une unité de calcul et une unité d'affichage, **caractérisé en ce que** l'unité d'enregistrement optique comprend une source lumineuse, un détecteur de lumière, un capteur de température et une unité de commande, laquelle produit les signaux de commande, et le système de mesure optique est conçu pour mettre en oeuvre le procédé de mesure optique selon l'une quelconque des revendications 1 à 9.
